# EUROPEAN PATENT APPLICATION

(11) **EP 0 892 053 A2**
(43) Date of publication of application: **20.01.1999**
(21) Application number: 98305066.7
(22) Date of filing: 26.06.1998
(51) Int. Cl.: C12N 15/16, C07K 14/575, C07K 16/26, A61K 48/00, C12Q 1/68

(54) **production of recombinant HPMBQ91 polypeptide and uses thereof**

(30) Priority: 14.07.1997 EP 97305215
(71) Applicant: SMITHKLINE BEECHAM PLC, Brentford, Middlesex TW8 9EP (GB); Human Genome Sciences, Rockville, Maryland 20850 (US)
(72) Inventor: Duckworth, David M., SmithKline Beecham Pharma., Harlow, Essex, CM19 5AW (GB); Hastings, Gregg A., Rockville, Maryland 20850 (US); Ruben, Steven M., Rockville, Maryland 20850 (US)
(74) Representative: Connell, Anthony Christopher

(57) **Abstract**

HPMBQ91 polypeptides and polynucleotides and methods for producing such polypeptides by recombinant techniques are disclosed. Also disclosed are methods for utilizing HPMBQ91 polypeptides and polynucleotides in the design of protocols for the treatment of neuronal disorders including pain, CNS related disorders, gastrointestinal disorders, cardiovascular disorders, metabolic disorders including diabetes and obesity, smooth muscle disorders, inflammatory disorders including asthma, cancers including adenomas, leiomyomas, liposarcomas, lipomas, melanomas, pulmonary chondroid hamartomas, lung cancer, prostate cancer and breast cancer, among others, and diagnostic assays for such conditions.

### Annex to the description

SEQUENCE LISTING

(1) GENERAL INFORMATION

(i) APPLICANT: SmithKline Beecham plc and Human Genome Sciences

(ii) TITLE OF THE INVENTION: Novel Compounds

(iii) NUMBER OF SEQUENCES: 4

(iv) CORRESPONDENCE ADDRESS:
(A) ADDRESSEE: SmithKline Beecham, Corporate Intellectual Property
(B) STREET: Two New Horizons Court
(C) CITY: Brentford
(D) STATE: Middlesex
(E) COUNTRY: United Kingdom
(F) ZIP: TW8 9EP

(v) COMPUTER READABLE FORM:
(A) MEDIUM TYPE: Diskette
(B) COMPUTER: IBM Compatible
(C) OPERATING SYSTEM: DOS
(D) SOFTWARE: FastSEQ for Windows Version 2.0

(2) INFORMATION FOR SEQ ID NO:1:

(i) SEQUENCE CHARACTERISTICS:
(A) LENGTH: 778 base pairs
(B) TYPE: nucleic acid

(C) STRANDEDNESS: single
(D) TOPOLOGY: linear

(ii) MOLECULE TYPE: cDNA

(xi) SEQUENCE DESCRIPTION: SEQ ID NO:1:

CTCCACTCGG TTTCTCTCTT TGCAGGAGCA CCGGCAGCAC CAGTGTGTGA GGGGAGCAGG 60
CAGCGGTCCT AGCCAGTTCC TTGATCCTGC CAGACCACCC AGCCCCTGGC ACAGAGCTGC 120
TCCACAGGCA CCATGAGGAT CATGCTGCTA TTCACAGCCA TCCTGGCCTT CAGCCTAGCT 180
CAGAGCTTTG GGGCTGTCTG TAAGGAGCCA CAGGAGGAGG TGGTTCCTGG CGGGGGCCGC 240
AGCAAGAGGG ATCCAGATCT CTACCAGCTG CTCCAGAGAC TCTTCAAAAG CCACTCATCT 300
CTGGAGGGAT TGCTCAAAGC CCTGAGCCAG GCTAGCACAG ATCCTAAGGA ATCAACATCT 360
CCCGAGAAAC GTGACATGCA TGACTTCTTT GTGGGACTTA TGGGCAAGAG GAGCGTCCAG 420
CCAGACTCTC CTACGGATGT GAATCAAGAG AACGTCCCCA GCTTTGGCAT CCTCAAGTAT 480
CCCCCGAGAG CAGAATAGGT ACTCCACTTC CGGACTCCTG GACTGCATTA GGAAGACCTC 540
TTTCCCTGTC CCAATCCCCA GGTGCGCACG CTCCTGTTAC CCTTTCTCTT CCCTGTTCTT 600
GTAACATTCT NGTGCTTTGA CTCCTTCTCC ATCTTTTCTA CCTGACCCTG GTGTGGAAAC 660
TGCATAGTGA ATATCCCCAA CCCCAATGGG CATTGACTGT AGAATACCCT AGAGTTCCTG 720
TAGTGTCCTA CATTAAAAAT ATAATGTCTC TCTCTATTCC TCAACAAATA AAGGATTT 778

(2) INFORMATION FOR SEQ ID NO:2:

(i) SEQUENCE CHARACTERISTICS:
(A) LENGTH: 121 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:2:

(2) INFORMATION FOR SEQ ID NO:3:

(i) SEQUENCE CHARACTERISTICS:
(A) LENGTH: 39 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:3:

Asp Pro Asp Leu Tyr Gln Leu Leu Gln Arg Leu Phe Lys Ser His Ser
1                   5                   10                  15
Ser Leu Glu Gly Leu Leu Lys Ala Leu Ser Gln Ala Ser Thr Asp Pro
            20                  25                  30
Lys Glu Ser Thr Ser Pro Glu

        35

(2) INFORMATION FOR SEQ ID NO:4:

(i) SEQUENCE CHARACTERISTICS:
(A) LENGTH: 10 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:4:

Asp Met His Asp Phe Phe Val Gly Leu Met
1               5                   10

## Description

### Field of the invention

This invention relates to newly identified polynucleotides, polypeptides encoded by them and to the use of such polynucleotides and polypeptides, and to their production. More particularly, the polynucleotides and polypeptides of the present invention relate to a neuropeptide precursor hereinafter referred to as HPMBQ91 and the peptide products thereof. The invention also relates to inhibiting or activating the action of such polynucleotides and polypeptides.

### Background of the invention

It is well established that neuropeptides are derived by proteolytic processing of a larger preprohormone precursor protein. Peptides within preprohormones are typically flanked by pairs of basic residues, for example, Lys-Arg (KR), Arg-Arg (RR), Lys-Lys (KK) or Arg-Lys (RK). Neuropeptides may also undergo post-translational modifications that include disulphide bond formation, glycosylation, C terminal alpha-amidation, phosphorylation and sulphation (V. Y. H. Hook *et al.,* FASEB J, 8: 1269-1278, 1994 and refs therein). It is not uncommon for the preprohormone processing to give rise to more than one neuropeptide, for example, pro-opiomelanocortin (H.Takahashi *et al.,* FEBS Lett., 1981, 135: 97-102). The cleaved, active neuropeptides usually exert their biological action by interacting with the membrane protein superfamily of G-protein coupled receptors (Lefkowitz, Nature, 1991, 351:353-354). G-protein coupled receptors have been characterized as including these seven conserved hydrophobic stretches of about 20 to 30 amino acids, connecting at least eight divergent hydrophilic loops. The G-protein family of coupled receptors includes dopamine receptors which bind to neuroleptic drugs used for treating psychotic and neurological disorders. Other examples of members of this family include, but are not limited to, calcitonin, adrenergic, endothelin, cAMP, adenosine, muscarinic, acetylcholine, serotonin, histamine, thrombin, kinin, follicle stimulating hormone, opsins, endothelial differentiation gene-1, rhodopsins, odorant, and cytomegalovirus receptors.

Neurokinin B (also known as neuromedin K) is a neuropeptide that is known to excite neurons and contract smooth muscle. Neurokinin B has been shown to be involved in vasodilation, feeding responses, and other behavioural responses. Whilst the mouse, bovine and rat orthologs have all been cloned and published (mouse neurokinin B precursor; K. Kako *et al.,* Biomed. Res. 14: 253-259, 1993; bovine neuromedin K precursor; K. H. Hoshimaru *et al.,* Proc. Natl. Acad. Sci. 83: 7074-7078, 1986; rat neurokinin B precursor; T. I. Bonner *et al.,* Brain Res. Mol. Brain Res. 2: 243-249, 1987), the human sequence has remained unknown until the disclosure of the present invention.

### Summary of the invention

In one aspect, the invention relates to HPMBQ91 polypeptides and recombinant materials and methods for their production. In a further aspect, the present invention relates to one or more peptides formed by enzymatic processing of HPMBQ91 polypeptides. Another aspect relates to using such HPMBQ91 polypeptides and polynucleotides and such peptides formed by enzymatic processing of HPMBQ91 polypeptides. Such uses include the treatment of neuronal disorders including pain, CNS related disorders, gastrointestinal disorders, cardiovascular disorders, metabolic disorders including diabetes and obesity, smooth muscle disorders, inflammatory disorders including asthma, cancers including adenomas, leiomyomas, liposarcomas, lipomas, melanomas, pulmonary chondroid hamartomas, lung cancer, prostate cancer and breast cancer, among others. In still another aspect, the invention relates to methods to identify agonists and antagonists using the materials provided by the invention, and treating conditions associated with HPMBQ91 imbalance with the identified compounds. Yet another aspect of the invention relates to diagnostic assays for detecting diseases associated with inappropriate HPMBQ91 activity or levels. Yet another aspect of the invention relates to diagnostic assays for detecting diseases associated with inappropriate activity or levels of peptides processed from HPMBQ91.

### Description of the invention

### Definitions

The following definitions are provided to facilitate understanding of certain terms used frequently herein.

"HPMBQ91 activity or HPMBQ91 polypeptide activity" or "biological activity of the HPMBQ91 or HPMBQ91 polypeptide" refers to the metabolic or physiological function of said HPMBQ91 polypeptide, including peptides derived therefrom. This definition includes similar activities or improved activities or these activities with decreased undesirable side-effects. Also included are antigenic and immunogenic activities of said HPMBQ91 polypeptides and peptides derived therefrom.

"Antibodies" as used herein includes polyclonal and monoclonal antibodies, chimeric, single chain, and humanized antibodies, as well as Fab fragments, including the products of an Fab or other immunoglobulin expression library.

"Isolated" means altered "by the hand of man" from the natural state. If an "isolated" composition or substance occurs in nature, it has been changed or removed from its original environment, or both. For example, a polynucleotide or a polypeptide naturally present in a living animal is not "isolated," but the same polynucleotide or polypeptide separated from the coexisting materials of its natural state is "isolated", as the term is employed herein.

"Polynucleotide" generally refers to any polyribonucleotide or polydeoxribonucleotide, which may be unmodified RNA or DNA or modified RNA or DNA. "Polynucleotides" include, without limitation single- and double-stranded DNA, DNA that is a mixture of single- and double-stranded regions, single- and double-stranded RNA, and RNA that is mixture of single-and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term polynucleotide also includes DNAs or RNAs containing one or more modified bases and DNAs or RNAs with backbones modified for stability or for other reasons. "Modified" bases include, for example, tritylated bases and unusual bases such as inosine. A variety of modifications has been made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

"Polypeptide" refers to any peptide or protein comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids. "Polypeptides" include amino acid sequences modified either by natural processes, such as posttranslational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent crosslinks, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. See, for instance, PROTEINS - STRUCTURE AND MOLECULAR PROPERTIES, 2nd Ed., T. E. Creighton, W. H. Freeman and Company, New York, 1993 and Wold, F., Posttranslational Protein Modifications: Perspectives and Prospects, pgs. 1-12 in POSTTRANSLATIONAL COVALENT MODIFICATION OF PROTEINS, B. C. Johnson, Ed., Academic Press, New York, 1983; Seifter *et al.,* "Analysis for protein modifications and nonprotein cofactors", *Meth Enzymol* (1990) 182:626-646 and Rattan *et al.,* "Protein Synthesis: Posttranslational Modifications and Aging", *Ann NY Acad Sci* (1992) 663:48-62.

"Variant" as the term is used herein, is a polynucleotide or polypeptide that differs from a reference polynucleotide or polypeptide respectively, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques or by direct synthesis.

"% identity", as known in the art, is a measure of the relationship between two polypeptide sequences or two polynucleotide sequences, as determined by comparing their sequences. In general, the two sequences to be compared are aligned to give a maximum correlation between the sequences. The alignment of the two sequences is examined and the number of positions giving an exact amino acid or nucleotide correspondence between the two sequences determined, divided by the total length of the alignment and multiplied by 100 to give a % identity figure. This % identity figure may be determined over the whole length of the sequences to be compared, which is particularly suitable for sequences of the same or very similar length and which are highly homologous, or over shorter defined lengths, which is more suitable for sequences of unequal length or which have a lower level of homology.

"% similarity", as known in the art, is a further measure of the relationship between two polypeptide sequences. The two sequences being compared are aligned to give maximum correlation between the sequences. The alignment of the two sequences is examined at each position and a score is determined according to the chemical and/or physical properties of the amino acids being compared at that position. Such chemical and/or physical properties include charge, size and hydrophobicity of the amino acid side chains.

Methods for comparing the identity and similarity of two or more sequences are well known in the art. Thus for instance, programs available in the Wisconsin Sequence Analysis Package, version 9.1 (Devereux J et al, Nucleic Acids Res, 12, 387-395, 1984, available from Genetics Computer Group, Madison, Wisconsin, USA), for example the programs BESTFIT and GAP, may be used to determine the % identity between two polynucleotides and the % identity and the % similarity between two polypeptide sequences. BESTFIT uses the "local homology" algorithm of Smith and Waterman (Advances in Applied Mathematics, 2, 482-489, 1981) and finds the best single region of similarity between two sequences. BESTFIT is more suited to comparing two polynucleotide or two polypeptide sequences which are dissimilar in length, the program assuming that the shorter sequence represents a portion of the longer. In comparison, GAP aligns two sequences, finding a "maximum similarity", according to the algorithm of Neddleman and
Wunsch (J Mol Biol, 48, 443-453, 1970). GAP is more suited to comparing sequences which are approximately the same length and an alignment is expected over the entire length. Preferably, the parameters "Gap Weight" and "Length Weight" used in each program are 50 and 3, for polynucleotide sequences and 12 and 4 for polypeptide sequences, respectively. Preferably, % identities and similarities are determined when the two sequences being compared are optimally aligned.

Other programs for determining identity and/or similarity between sequences are also known in the art, for instance the BLAST family of programs (Altschul S F et al, J Mol Biol, 215, 403-410, 1990, Altschul S F et al, Nucleic Acids Res., 25:389-3402, 1997, available from the National Center for Biotechnology Information (NCBI), Bethesda, Maryland, USA and accessible through the home page of the NCBI at www.ncbi.nlm.nih.gov) and FASTA (Pearson W R and Lipman D J, Proc Nat Acad Sci USA, 85, 2444-2448,1988, available as part of the Wisconsin Sequence Analysis Package). Preferably, the BLOSUM62 amino acid substitution matrix (Henikoff S and Henikoff J G, Proc. Nat. Acad Sci. USA, 89, 10915-10919, 1992) is used in polypeptide sequence comparisons including where nucleotide sequences are first translated into amino acid sequences before comparison.

Preferably, the program BESTFIT is used to determine the % identity of a query polynucleotide or a polypeptide sequence with respect to a polynucleotide or a polypeptide sequence of the present invention, the query and the reference sequence being optimally aligned and the parameters of the program set at the default value.

### Polypeptides of the Invention

In one aspect, the present invention relates to HPMBQ91 polypeptides. The HPMBQ91 polypeptides include the polypeptide of SEQ ID NO:2; as well as polypeptides comprising the amino acid sequence of SEQ ID NO: 2; and polypeptides comprising the amino acid sequence which have at least 80% identity to that of SEQ ID NO:2 over its entire length, and still more preferably at least 90% identity, and even still more preferably at least 95% identity to SEQ ID NO: 2. Furthermore, those with at least 97-99% are highly preferred. Also included within HPMBQ91 polypeptides are polypeptides having the amino acid sequence which have at least 80% identity to the polypeptide having the amino acid sequence of SEQ ID NO:2 over its entire length, and still more preferably at least 90% identity, and still more preferably at least 95% identity to SEQ ID NO:2. Furthermore, those with at least 97-99% are highly preferred.

The polypeptide of SEQ ID NO:2 is believed to be the human neurokinin B (neuromedin K) precursor. SEQ ID NO:1 is the cDNA sequence of the human neurokinin B precursor and SEQ ID NO:2 is the amino acid sequence of the human neurokinin B precursor polypeptide. The precursor has a putative signal sequence from amino acid residues 1 to 20 which is believed to be cleaved between glycine 20 and alanine 21 based on similarity with the mouse, rat and bovine sequences.

Preferably the HPMBQ91 polypeptide exhibits at least one biological activity of HPMBQ91.

The HPMBQ91 polypeptides may be in the form of the "mature" protein or may be a part of a larger protein such as a precursor or fusion protein. It is often advantageous to include an additional amino acid sequence which contains secretory or leader sequences, pro-sequences, sequences which aid in purification such as multiple histidine residues, or an additional sequence for stability during recombinant production.

In a further aspect, the present invention relates to HPMBQ91 peptides. Such peptides are enzymatically processed from HPMBQ91 and include, but are not limited to, the amino acid sequence of SEQ ID NO:3, as well as peptides comprising the amino acid sequence of SEQ ID NO: 3; and peptides comprising the amino acid sequence which have at least 80% identity to that of SEQ ID NO:3 over its entire length, and still more preferably at least 90% identity, and even still more preferably at least 95% identity to SEQ ID NO: 3. Furthermore, those with at least 97-99% are highly preferred. Also included within the HPMBQ91 peptides are peptides having the amino acid sequence which have at least 80% identity to the polypeptide having the amino acid sequence of SEQ ID NO:3 over its entire length, and still more preferably at least 90% identity, and still more preferably at least 95% identity to SEQ ID NO:3. Furthermore, those with at least 97-99% are highly preferred.

SEQ ID NO:3 is the amino acid sequence of a novel peptide derived from the human neurokinin B precursor. The sequence of the peptide given as SEQ ID NO:3 has been deduced from the position of putative N-terminal and C-terminal peptide cleavage sites (KR and KR respectively).

A further peptide is the known peptide of SEQ ID NO:4, human neurokinin B peptide. The C-terminal glycine in the neurokinin B cleavage product functions as an amide donor to the adjacent methionine and does not form part of the final active peptide. Preferably HPMBQB1 peptides exhibit at least one biological activity of HPMBQ91.

Fragments of the HPMBQ91 polypeptides are also included in the invention. A fragment is a polypeptide having an amino acid sequence that is the same as part, but not all, of the amino acid sequence of the aforementioned HPMBQ91 polypeptides. As with HPMBQ91 polypeptides, fragments may be "free-standing," or comprised within a larger polypeptide of which they form a part or region, most preferably as a single continuous region. Representative examples of polypeptide fragments of the invention, include, for example, fragments from about amino acid number 1-20, 21-40, 41-60, 61-80, 81-100, and 101 to the end of HPMBQ91 polypeptide. In this context "about" includes the particularly recited ranges larger or smaller by several, 5, 4, 3, 2 or 1 amino acid at either extreme or at both extremes.

Preferred fragments include, for example, truncation polypeptides having the amino acid sequence of HPMBQ91 polypeptides, except for deletion of a continuous series of residues that includes the amino terminus, or a continuous series of residues that includes the carboxyl terminus or deletion of two continuous series of residues, one including the amino terminus and one including the carboxyl terminus. Also preferred are fragments characterized by structural or functional attributes such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Other preferred fragments are biologically active fragments. Biologically active fragments are those that mediate HPMBQ91 activity, including those with a similar activity or an improved activity, or with a decreased undesirable activity. Also included are those that are antigenic or immunogenic in an animal, especially in a human.

Preferably, all of these polypeptide fragments retain the biological activity of the HPMBQ91, including antigenic activity. Variants of the defined sequence and fragments also form part of the present invention. Preferred variants are those that vary from the referents by conservative amino acid substitutions -- i.e., those that substitute a residue with another of like characteristics. Typical such substitutions are among Ala, Val, Leu and Ile; among Ser and Thr; among the acidic residues Asp and Glu; among Asn and Gln; and among the basic residues Lys and Arg; or aromatic residues Phe and Tyr. Particularly preferred are variants in which several, 5-10, 1-5, or 1-2 amino acids are substituted, deleted, or added in any combination.

The HPMBQ91 polypeptides and peptides of the invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides and peptides are well understood in the art.

### Polynucleotides of the Invention

Another aspect of the invention relates to HPMBQ91 polynucleotides. HPMBQ91 polynucleotides include isolated polynucleotides which encode the HPMBQ91 polypeptides and fragments, and polynucleotides closely related thereto. More specifically HPMBQ91 polynucleotides include a polynucleotide comprising a nucleotide sequence that has at least 85% identity to a nucleotide sequence encoding the HPMBQ91 polypeptide of SEQ ID NO:2 over its entire length, and a polynucleotide that is at least 85% identical to that of SEQ ID NO:1 over its entire length. In this regard, polynucleotides at least 90% identical are particularly preferred, and those with at least 95% are especially preferred. Furthermore, those with at least 97% are highly preferred and those with at least 98-99% are most highly preferred, with at least 99% being the most preferred. HPMBQ91 polynucleotides also include the polynucleotide having the particular sequence of SEQ ID NO:1 and the HPMBQ91 polypeptide encoding sequence contained in SEQ ID NO:1. Also included under HPMBQ91 polynucleotides are a nucleotide sequence which has sufficient identity to a nucleotide sequence contained in SEQ ID NO: 1 or contained in the cDNA insert in the plasmid deposited with the ATCC Deposit number 97375 to hybridize under conditions useable for amplification or for use as a probe or marker. Moreover, HPMBQ91 polynucleotide include a nucleotide sequence having at least 80% identity to a nucleotide sequence encoding the HPMBQ91 polypeptide expressed by the cDNA insert deposited at the ATCC with Deposit Number 97375, and a nucleotide sequence comprising at least 15 contiguous nucleotides of such cDNA insert. In this regard, polynucleotides at least 90% identical are particularly preferred, and those with at least 95% are especially preferred. Furthermore, those with at least 97% are highly preferred and those with at least 98-99% are most highly preferred, with at least 99% being the most preferred. The invention also provides polynucleotides which are complementary to all the above HPMBQ91 polynucleotides.

A deposit containing a human HPMBQ91 cDNA has been deposited with the American Type Culture Collection (ATCC), 12301 Park Lawn Drive, Rockville, Maryland 20852, USA, on December 8, 1995, and assigned ATCC Deposit Number 97375. The deposited material (clone) contains the full length HPMBQ91 cDNA, referred to as "DNA plasmid, 769547 (HPMBQ91)" upon deposit. The nucleotide sequence of the polynucleotides contained in the deposited material, as well as the amino acid sequence of the polypeptide encoded thereby, are controlling in the event of any conflict with any description of sequences herein.

The deposit has been made under the terms of the Budapest Treaty on the international recognition of the deposit of micro-organisms for purposes of patent procedure. The strain will be irrevocably and without restriction or condition released to the public upon the issuance of a patent. The deposit is provided merely as convenience to those of skill in the art and is not an admission that a deposit is required for enablement, such as that required under 35 U.S.C. §112.

HPMBQ91 of the invention is structurally related to other neuropeptides, as shown by the results of sequencing the cDNA of SEQ ID NO:1 encoding human HPMBQ91. The cDNA sequence of SEQ ID NO:1 contains an open reading frame (nucleotide number 1 to 360) encoding a polypeptide of 121 amino acids of SEQ ID NO:2. Amino acid sequence of SEQ ID NO:2 shows homology to mouse neurokinin B precursor (K. Kako *et al.,* Biomed. Res. 14: 253-259, 1993), bovine neuromedin K precursor (K. H. Hoshimaru *et al.,* Proc.Natl.Acad.Sci. 83: 7074-7078, 1986) and rat neurokinin B precursor (T. I. Bonner *et al.,* Brain Res. Mol. Brain Res. 2: 243-249, 1987). The nucleotide sequence of SEQ ID NO:1 shows homology to mouse neurokinin B precursor (K. Kako *et al.,* Biomed. Res. 14: 253-259, 1993), bovine neuromedin K precursor (K. H. Hoshimaru *et al.,* Proc.Natl.Acad.Sci. 83: 7074-7078, 1986) and rat neurokinin B precursor (T. I. Bonner *et al.,* Brain Res. Mol. Brain Res. 2: 243-249, 1987).

One polynucleotide of the present invention encoding HPMBQ91 may be obtained using standard cloning and screening, from a cDNA library derived from mRNA in cells of human placenta, fetal liver spleen, lymph node merkel cells using the expressed sequence tag (EST) analysis (Adams, M.D., *et al. Science* (1991) 252:1651-1656; Adams, M.D. *et al., Nature,* (1992) *355*:632-634; Adams, M.D., *et al., Nature* (1995) 377 Supp:3-174). Polynucleotides of the invention can also be obtained from natural sources such as genomic DNA libraries or can be synthesized using well known and commercially available techniques.

The nucleotide sequence encoding HPMBQ91 polypeptide of SEQ ID NO:2 may be identical to the polypeptide encoding sequence of SEQ ID NO: 1 (nucleotide number 1 to 360) or it may be a sequence, which as a result of the redundancy (degeneracy) of the genetic code, also encodes the polypeptide of SEQ ID NO:2.

When the polynucleotides of the invention are used for the recombinant production of HPMBQ91 polypeptide, the polynucleotide may include the coding sequence for the mature polypeptide or a fragment thereof, by itself; the coding sequence for the mature polypeptide or fragment in reading frame with other coding sequences, such as those encoding a leader or secretory sequence, a pre-, or pro- or prepro- protein sequence, or other fusion peptide portions. For example, a marker sequence which facilitates purification of the fused polypeptide can be encoded. In certain preferred embodiments of this aspect of the invention, the marker sequence is a hexahistidine peptide, as provided in the pQE vector (Qiagen, Inc.) and described in Gentz *et al., Proc Natl Acad Sci USA* (1989) 86:821-824, or is an HA tag. The polynucleotide may also contain noncoding 5' and 3' sequences, such as transcribed, non-translated sequences, splicing and polyadenylation signals, ribosome binding sites and sequences that stabilize mRNA.

Further preferred embodiments are polynucleotides encoding HPMBQ91 variants comprising the amino acid sequence of SEQ ID NO:2, in which several, 5-10, 1-5, 1-3, 1-2 or 1 amino acid residues are substituted, deleted or added, in any combination.

The present invention further relates to polynucleotides that hybridize to the herein above-described sequences. In this regard, the present invention especially relates to polynucleotides which hybridize under stringent conditions to the herein above-described polynucleotides. As herein used, the term "stringent conditions" means hybridization will occur only if there is at least 95% and preferably at least 97% identity between the sequences.

Polynucleotides of the invention, which are identical or sufficiently identical to a nucleotide sequence contained in SEQ ID NO: 1 or a fragment thereof, or to the cDNA insert in the plasmid deposited at the ATCC with Deposit Number 97375 or a fragment thereof, may be used as hybridization probes for cDNA and genomic DNA, to isolate full-length cDNAs and genomic clones encoding HPMBQ91 polypeptide and to isolate cDNA and genomic clones of other genes that have a high sequence similarity to the HPMBQ91 gene. Such hybridization techniques are known to those of skill in the art. Typically these nucleotide sequences are 80% identical, preferably 90% identical, more preferably 95% identical to that of the referent. The probes generally will comprise at least 15 nucleotides. Preferably, such probes will have at least 30 nucleotides and may have at least 50 nucleotides. Particularly preferred probes will range between 30 and 50 nucleotides.

A polynucleotide encoding the HPMBQ91 polypeptide may be obtained by screening an appropriate library under stingent hybridization conditions with a labeled probe having the SEQ ID NO: 1 or a fragment thereof; and isolating full-length cDNA and genomic clones containing said polynucleotide sequence. Thus in another aspect, HPMBQ91 polynucleotides of the present invention further include a nucleotide sequence comprising a nucleotide sequence that hybridizes under stringent condition to a nucleotide sequence having SEQ ID NO: 1 or a fragment thereof. In addition, HPMBQ91 polypeptides also include peptides comprising amino acid sequences encoded by the nucleotide sequence obtained by the above hybridization condition. Such hybridization techniques are well known to those of skill in the art. Stringent hybridization conditions are as defined above or alternatively conditions under overnight incubation at 42°C in a solution comprising: 50% formamide, 5xSSC (150mM NaCl, 15mM trisodium citrate), 50 mM sodium phosphate (pH7.6), 5x Denhardt's solution, 10 % dextran sulfate, and 20 microgram/ml denatured, sheared salmon sperm DNA, followed by washing the filters in 0.1x SSC at about 65°C.

The polynucleotides and polypeptides of the present invention may be employed as research reagents and materials for discovery of treatments and diagnostics to animal and human disease.

### Vectors, Host Cells, Expression

The present invention also relates to vectors which comprise a polynucleotide or polynucleotides of the present invention, and host cells which are genetically engineered with vectors of the invention and to the production of polypeptides of the invention by recombinant techniques. Cell-free translation systems can also be employed to produce such proteins using RNAs derived from the DNA constructs of the present invention.

For recombinant production, host cells can be genetically engineered to incorporate expression systems or portions thereof for polynucleotides of the present invention. Introduction of polynucleotides into host cells can be effected by methods described in many standard laboratory manuals, such as Davis et al., *BASIC METHODS IN MOLECULAR BIOLOGY*(1986) and Sambrook et al., *MOLECULAR CLONING: A LABORATORY MANUAL,* 2nd Ed., Cold Spring Harbor Laboratory Press, Cold Spring Harbor, N.Y. (1989) such as calcium phosphate transfection, DEAE-dextran mediated transfection, transvection, microinjection, cationic lipid-mediated transfection, electroporation, transduction, scrape loading, ballistic introduction or infection.

Representative examples of appropriate hosts include bacterial cells, such as Streptococci, Staphylococci, *E. coli, Streptomyces* and *Bacillus subtilis* cells; fungal cells, such as yeast cells and *Aspergillus* cells; insect cells such as *Drosophila* S2 and *Spodoptera* Sf9 cells; animal cells such as CHO, COS, HeLa, C127, 3T3, BHK, HEK 293 and Bowes melanoma cells; and plant cells.

A great variety of expression systems can be used. Such systems include, among others, chromosomal, episomal and virus-derived systems, e.g., vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids. The expression systems may contain control regions that regulate as well as engender expression. Generally, any system or vector suitable to maintain, propagate or express polynucleotides to produce a polypeptide in a host may be used. The appropriate nucleotide sequence may be inserted into an expression system by any of a variety of well-known and routine techniques, such as, for example, those set forth in Sambrook *et al., MOLECULAR CLONING, A LABORATORY MANUAL* (*supra*).

For secretion of the translated protein into the lumen of the endoplasmic reticulum, into the periplasmic space or into the extracellular environment, appropriate secretion signals may be incorporated into the desired polypeptide. These signals may be endogenous to the polypeptide or they may be heterologous signals.

The HPMBQ91 polypeptide may be expressed for use in screening assays. The polypeptide may be produced at the surface of the cell in which case the cells may be harvested prior to use in the screening assay. Alternatively the HPMBQ91 polypeptide or polypeptides are secreted into the medium, and the medium then recovered in order to recover and purify the polypeptide or polypeptides. If produced intracellularly, the cells must first be lysed before the polypeptide or polypeptides are recovered.

HPMEQ91 polypeptides can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography is employed for purification. Well known techniques for refolding proteins may be employed to regenerate active conformation when the polypeptide is denatured during isolation and or purification.

### Diagnostic Assays

This invention also relates to the use of HPMBQ91 polynucleotides for use as diagnostic reagents. Detection of a mutated fonn of HPMBQ91 gene associated with a dysfunction will provide a diagnostic tool that can add to or define a diagnosis of a disease or susceptibility to a disease which results from under-expression, over-expression or altered expression of HPMBQ91. Individuals carrying mutations in the HPMBQ91 gene may be detected at the DNA level by a variety of techniques.

Nucleic acids for diagnosis may be obtained from a subject's cells, such as from blood, urine, saliva, tissue biopsy or autopsy material. The genomic DNA may be used directly for detection or may be amplified enzymatically by using PCR or other amplification techniques prior to analysis. RNA or cDNA may also be used in similar fashion. Deletions and insertions can be detected by a change in size of the amplified product in comparison to the normal genotype. Point mutations can be identified by hybridizing amplified DNA to labeled HPMBQ91 nucleotide sequences. Perfectly matched sequences can be distinguished from mismatched duplexes by RNase digestion, by differences in melting temperatures or by HPLC. DNA sequence differences may also be detected by alterations in electrophoretic mobility of DNA fragments in gels, with or without denaturing agents, or by direct DNA sequencing. See, e.g., Myers *et al., Science* (1985) 230:1242. Sequence changes at specific locations may also be revealed by nuclease protection assays, such as RNase and S1 protection or the chemical cleavage method. See Cotton *et al., Proc Natl Acad Sci USA* (1985) 85: 4397-4401. In another embodiment, an array of oligonucleotides probes comprising HPMBQ91 nucleotide sequence or fragments thereof can be constructed to conduct efficient screening of e.g., genetic mutations. Array technology methods are well known and have general applicability and can be used to address a variety of questions in molecular genetics including gene expression, genetic linkage, and genetic variability. (See for example: M.Chee et al., Science, Vol 274, pp 610-613 (1996)).

The diagnostic assays offer a process for diagnosing or determining a susceptibility to neuronal disorders including pain, CNS related disorders, gastrointestinal disorders, cardiovascular disorders, metabolic disorders including diabetes and obesity, smooth muscle disorders, inflammatory disorders including asthma, cancers including adenomas, leiomyomas, liposarcomas, lipomas, melanomas, pulmonary chondroid hamartomas, lung cancer, prostate cancer and breast cancer, among others through detection of mutation in the HPMBQ91 gene by the methods described.

In addition, neuronal disorders including pain, CNS related disorders, gastrointestinal disorders, cardiovascular disorders, metabolic disorders including diabetes and obesity, smooth muscle disorders, inflammatory disorders including asthma, cancers including adenomas, leiomyomas, liposarcomas, lipomas, melanomas, pulmonary chondroid hamartomas, lung cancer, prostate cancer and breast cancer, among others, can be diagnosed by methods comprising detennining from a sample derived from a subject an abnormally decreased or increased level of HPMBQ91 polypeptide or HPMBQ91 mRNA. Decreased or increased expression can be measured at the RNA level using any of the methods well known in the art for the quantitation of polynucleotides, such as, for example, PCR, RT-PCR, RNase protection, Northern blotting and other hybridization methods. Assay techniques that can be used to determine levels of a protein, such as an HPMBQ91 polypeptide, in a sample derived from a host are well-known to those of skill in the art. Such assay methods include radioimmunoassays, competitive-binding assays, Western Blot analysis and ELISA assays.

### Chromosome Assays

The nucleotide sequences of the present invention are also valuable for chromosome localisation. The sequence is specifically targeted to, and can hybridize with, a particular location on an individual human chromosome. The mapping of relevant sequences to chromosomes according to the present invention is an important first step in correlating those sequences with gene associated disease. Once a sequence has been mapped to a precise chromosomal location, the physical position of the sequence on the chromosome can be correlated with genetic map data. Such data are found in, for example, V. McKusick, Mendelian Inheritance in Man (available on-line through Johns Hopkins University Welch Medical Library). The relationship between genes and diseases that have been mapped to the same chromosomal region are then identified through linkage analysis (coinheritance of physically adjacent genes).

The HPMBQ91 gene has been mapped to chromosome 12 between the markers D12S1632 and D12S355, which corresponds to 12q14. There is a breakpoint region at 12q14-15, and consequently a high incidence of chromosomal abberration and links to various cancers.

The differences in the cDNA or genomic sequence between affected and unaffected individuals can also be determined. If a mutation is observed in some or all of the affected individuals but not in any normal individuals, then the mutation is likely to be the causative agent of the disease.

The nucleotide sequences of the present invention are also valuable for tissue localisation. Such techniques allow the determination of expression patterns of the HPMBQ91 polypeptides in tissues by detection of the mRNAs that encode them. These techniques include in situ hybridziation techniques and nucleotide amplification techniques, for example PCR. Such techniques are well known in the art. Results from these studies provide an indication of the normal functions of the polypeptides in the organism. In addition, comparative studies of the normal expression pattern of HPMBQ91 mRNAs with that of mRNAs encoded by a HPMBQ91 gene provide valuable insights into the role of mutant HPMBQ91 polypeptides, or that of inappropriate expression of normal HPMBQ91 polypeptides, in disease. Such inappropriate expression may be of a temporal, spatial or simply quantitative nature.

### Antibodies

The polypeptides and peptides of the invention or their fragments or analogs thereof, or cells expressing them can also be used as immunogens to produce antibodies immunospecific for the HPMBQ91 polypeptides. The term "immunospecific" means that the antibodies have substantiall greater affinity for the polypeptides of the invention than their affinity for other related polypeptides in the prior art.

Antibodies generated against the HPMBQ91 polypeptides and peptides can be obtained by administering the polypeptides or epitope-bearing fragments, analogs or cells to an animal, preferably a nonhuman, using routine protocols. For preparation of monoclonal antibodies, any technique which provides antibodies produced by continuous cell line cultures can be used. Examples include the hybridoma technique (Kohler, G. and Milstein, C., *Nature* (1975) 256:495-497), the trioma technique, the human B-cell hybridoma technique (Kozbor *et al., Immunology Today* (1983) 4:72) and the EBV-hybridoma technique (Cole *et al.,* MONOCLONAL ANTIBODIES AND CANCER THERAPY, pp. 77-96, Alan R. Liss, Inc., 1985).

Techniques for the production of single chain antibodies (U.S. Patent No. 4,946,778) can also be adapted to produce single chain antibodies to polypeptides of this invention. Also, transgenic mice, or other organisms including other mammals, may be used to express humanized antibodies.

The above-described antibodies may be employed to isolate or to identify clones expressing the polypeptide or to purify the polypeptides by affinity chromatography.

Antibodies against HPMBQB1 polypeptides may also be employed to treat neuronal disorders including pain, CNS related disorders, gastrointestinal disorders, cardiovascular disorders, metabolic disorders including diabetes and obesity, smooth muscle disorders, inflammatory disorders including asthma, cancers including adenomas, leiomyomas, liposarcomas, lipomas, melanomas, pulmonary chondroid hamartomas, lung cancer, prostate cancer and breast cancer, among others.

### Vaccines

Another aspect of the invention relates to a method for inducing an immunological response in a mammal which comprises inoculating the mammal with HPMBQ91 polypeptide or peptides, or a fragment thereof, adequate to produce antibody and/or T cell immune response to protect said animal from neuronal disorders including pain, CNS related disorders, gastrointestinal disorders, cardiovascular disorders, metabolic disorders including diabetes and obesity, smooth muscle disorders, inflammatory disorders including asthma, cancers including adenomas, leiomyomas, liposarcomas, lipomas, melanomas, pulmonary chondroid hamartomas, lung cancer, prostate cancer and breast cancer, among others. Yet another aspect of the invention relates to a method of inducing immunological response in a mammal which comprises, delivering HPMBQ91 polypeptide or peptides via a vector directing expression of HPMBQ91 polynucleotide *in vivo* in order to induce such an immunological response to produce antibody to protect said animal from diseases.

A further aspect of the invention relates to an immunological/vaccine formulation (composition) which, when introduced into a mammalian host, induces an immunological response in that mammal to a HPMBQ91 polypeptide or peptides wherein the composition comprises a HPMBQ91 polypeptide or peptide or HPMBQ91 gene. The vaccine formulation may further comprise a suitable carrier. Since HPMBQ91 polypeptide or peptides may be broken down in the stomach, it is preferably administered parenterally (including subcutaneous, intramuscular, intravenous, intradermal etc. injection). Formulations suitable for parenteral administration include aqueous and non-aqueous sterile injection solutions which may contain anti-oxidants, buffers, bacteriostats and solutes which render the formulation instonic with the blood of the recipient; and aqueous and non-aqueous sterile suspensions which may include suspending agents or thickening agents. The formulations may be presented in unit-dose or multi-dose containers, for example, sealed ampoules and vials and may be stored in a freeze-dried condition requiring only the addition ofthe sterile liquid carrier immediately prior to use. The vaccine formulation may also include adjuvant systems for enhancing the immunogenicity of the formulation, such as oil-in water systems and other systems known in the art. The dosage will depend on the specific activity of the vaccine and can be readily determined by routine experimentation.

### Screening Assays

The HPMBQ91 polypeptide and peptides of the present invention may be employed in a screening process for compounds which activate (agonists) or inhibit activation of (antagonists, or otherwise called inhibitors) the HPMBQ91 polypeptide or peptides of the present invention. Thus, polypeptides or peptides of the invention may also be used to assess identify agonist or antagonists from, for example, cells, cell-free preparations, chemical libraries, and natural product mixtures. These agonists or antagonists may be natural substrates, ligands, receptors, etc., as the case may be, of the polypeptide or peptides of the present invention; or may be structural or functional mimetics of the polypeptide of the present invention. See Coligan *et al., Current Protocols in Immunology* 1(2):Chapter 5 (1991).

HPMBQ91 polypeptides and peptides are responsible for one or more biological functions, including one or more pathologies. Accordingly, it is desirous to find compounds and drugs which stimulate HPMBQ91 polypeptide or peptides on the one hand and which can inhibit the function of HPMBQ91 polypeptide or peptides on the other hand. In general, agonists are employed for therapeutic and prophylactic purposes for such conditions as neuronal disorders including pain, CNS related disorders, gastrointestinal disorders, cardiovascular disorders, metabolic disorders including diabetes and obesity, smooth muscle disorders, inflammatory disorders including asthma, cancers including adenomas, leiomyomas, liposarcomas, lipomas, melanomas, pulmonary chondroid hamartomas, lung cancer, prostate cancer and breast cancer, among others. Antagonists may be employed for a variety oftherapeutic and prophylactic purposes for such conditions as neuronal disorders including pain, CNS related disorders, gastrointestinal disorders, cardiovascular disorders, metabolic disorders including diabetes and obesity, smooth muscle disorders, inflammatory disorders including asthma, cancers including adenomas, leiomyomas, liposarcomas, lipomas, melanomas, pulmonary chondroid hamartomas, lung cancer, prostate cancer and breast cancer, among others.

In general, such screening procedures may involve using appropriate cells which express the HPMBQ91 polypeptide or respond to HPMBQ91 polypeptide of the present invention. Such cells include cells from mammals, yeast, *Drosophila* or *E. coli.* Cells which express the HPMBQ91 polypeptide (or cell membrane containing the expressed polypeptide) or respond to HPMBQ91 polypeptide are then contacted with a test compound to observe binding, or stimulation or inhibition of a functional response. The ability of the cells which were contacted with the candidate compounds is compared with the same cells which were not contacted for HPMBQ91 activity.

The assays may simply test binding of a candidate compound wherein adherence to the cells bearing the HPMBQ91 polypeptide is detected by means of a label directly or indirectly associated with the candidate compound or in an assay involving competition with a labeled competitor. Further, these assays may test whether the candidate compound results in a signal generated by activation of the HPMBQ91 polypeptide, using detection systems appropriate to the cells bearing the HPMBQ91 polypeptide. Inhibitors of activation are generally assayed in the presence of a known agonist and the effect on activation by the agonist by the presence of the candidate compound is observed. Standard methods for conducting such screening assays are well understood in the art.

Examples of potential HPMBQ91 polypeptide or peptide antagonists include antibodies or, in some cases, oligonucleotides or proteins which are closely related to the ligands, substrates, receptors, etc., as the case may be, of the HPMBQ91 polypeptide, e.g., a fragment of the ligands, substrates, receptors, or small molecules which bind to the polypeptide of the present invention but do not elicit a response, so that the activity of the polypeptide is prevented.

### Prophylactic and Therapeutic Methods

This invention provides methods of treating abnormal conditions such as neuronal disorders including pain, CNS related disorders, gastrointestinal disorders, cardiovascular disorders, metabolic disorders including diabetes and obesity, smooth muscle disorders, inflammatory disorders including asthma, cancers including adenomas, leiomyomas, liposarcomas, lipomas, melanomas, pulmonary chondroid hamartomas, lung cancer, prostate cancer and breast cancer, among others., related to both an excess of and insufficient amounts of HPMBQ91 polypeptide activity.

If the activity of HPMBQ91 polypeptide or peptides is in excess, several approaches are available. One approach comprises administering to a subject an inhibitor compound (antagonist) as hereinabove described along with a pharmaceutically acceptable carrier in an amount effective to inhibit the function of the HPMBQ91 polypeptide, such as, for example, by blocking the binding of ligands, substrates, etc., or by inhibiting a second signal, and thereby alleviating the abnormal condition. In another approach, soluble forms of HPMBQ91 polypeptides still capable of binding the ligand, substrate, etc. in competition with endogenous HPMBQ91 polypeptide may be administered. Typical embodiments of such competitors comprise fragments of the HPMBQ91 polypeptide.

In still another approach, expression of the gene encoding endogenous HPMBQ91 polypeptide can be inhibited using expression blocking techniques. Known such techniques involve the use of antisense sequences, either internally generated or externally administered (see, for example, O'Connor, J Neurochem (1991) 56:560 in Oligodeoxynucleotides as Antisense Inhibitors of Gene Expression, CRC Press, Boca Raton, FL (1988)). Alternatively, oligonucleotides which form triple helices ("triplexes") with the gene can be supplied (see, for example, Lee *et al.,* Nucleic Acids Res (1979) 6:3073; Cooney *et al.,* Science (1988) 241:456; Dervan *et al.,* Science (1991) 251:1360). These oligomers can be administered *per se* or the relevant oligomers can be expressed *in vivo.* Synthetic antisense or triplex oligonucleotides may comprise modified bases or modified backbones. Examples of the latter include methylphosphonate, phosphorothioate or peptide nucleic acid backbones. Such backbones are incorporated in the antisense or triplex oligonucleotide in order to provide protection from degradation by nucleases and are well known in the art. Antisense and triplex molecules synthesised with these or other modified backbones also form part of the present invention.

In addition, expression of the HPMBQ91 polypeptide may be prevented by using ribozymes specific to the HPMBQ91 mRNA sequence. Ribozymes are catalytically active RNAs that can be natural or synthetic (see for example Usman, N, et al., Curr. Opin. Struct. Biol (1996) 6(4), 527-33.) Synthetic ribozymes can be designed to specifically cleave HPMBQ91 mRNAs at selected positions thereby preventing translation of the HPMBQ91 mRNAs into functional polypeptide. Ribozymes may be synthesised with a natural ribose phosphate backbone and natural bases, as normally found in RNA molecules. Alternatively the ribosymes may be synthesised with non-natural backbones to provide protection from ribonuclease degradation, for example, 2'-O-methyl RNA, and may contain modified bases.

For treating abnormal conditions related to an under-expression of HPMBQ91 and its activity, several approaches are also available. One approach comprises administering to a subject a therapeutically effective amount of a compound which activates HPMBQ91 polypeptide, i.e., an agonist as described above, in combination with a pharmaceutically acceptable carrier, to thereby alleviate the abnormal condition. Alternatively, gene therapy may be employed to effect the endogenous production of HPMBQ91 by the relevant cells in the subject. For example, a polynucleotide of the invention may be engineered for expression in a replication defective retroviral vector, as discussed above. The retroviral expression construct may then be isolated and introduced into a packaging cell transduced with a retroviral plasmid vector containing RNA encoding a polypeptide of the present invention such that the packaging cell now produces infectious viral particles containing the gene of interest. These producer cells may be administered to a subject for engineering cells *in vivo* and expression of the polypeptide *in vivo.* For overview of gene therapy, see Chapter 20, *Gene Therapy and other Molecular Genetic-based Therapeutic Approaches,* (and references cited therein) in Human Molecular Genetics, T Strachan and A P Read, BIOS Scientific Publishers Ltd (1996). Another approach is to administer therapeutic amount of HPMBQ91 polypeptides in combination with a suitable pharmaceutical carrier.

### Formulation and Administration

HPMBQ91 polypeptides and peptides, agonists and antagonist peptides or small molecules, may be formulated in combination with a suitable pharmaceutical carrier. Such formulations comprise a therapeutically effective amount of the polypeptide or compound, and a pharmaceutically acceptable carrier or excipient. Such carriers include but are not limited to, saline, buffered saline, dextrose, water, glycerol, ethanol, and combinations thereof. Formulation should suit the mode of administration, and is well within the skill of the art. The invention further relates to pharmaceutical packs and kits comprising one or more containers filled with one or more of the ingredients of the aforementioned compositions of the invention.

Polypeptides and other compounds of the present invention may be employed alone or in conjunction with other compounds, such as therapeutic compounds.

Preferred forms of systemic administration of the pharmaceutical compositions include injection, typically by intravenous injection. Other injection routes, such as subcutaneous, intramuscular, or intraperitoneal, can be used. Alternative means for systemic administration include transmucosal and transdermal administration using penetrants such as bile salts or fusidic acids or other detergents. In addition, if properly formulated in enteric or encapsulated formulations, oral administration may also be possible. Administration of these compounds may also be topical and/or localized, in the form of salves, pastes, gels and the like.

The dosage range required depends on the choice of peptide, the route of administration, the nature of the formulation, the nature of the subject's condition, and the judgment of the attending practitioner. Suitable dosages, however, are in the range of 0.1-100 µg/kg of subject. Wide variations in the needed dosage, however, are to be expected in view of the variety of compounds available and the differing efficiencies of various routes of administration. For example, oral administration would be expected to require higher dosages than administration by intravenous injection. Variations in these dosage levels can be adjusted using standard empirical routines for optimization, as is well understood in the art.

Polypeptides used in treatment can also be generated endogenously in the subject, in treatment modalities often referred to as "gene therapy" as described above. Thus, for example, cells from a subject may be engineered with a polynucleotide, such as a DNA or RNA, to encode a polypeptide *ex vivo,* and for example, by the use of a retroviral plasmid vector. The cells are then introduced into the subject.

The example below was carried out using standard techniques, which are well known and routine to those of skill in the art, except where otherwise described in detail. The example illustrates, but does not limit the invention.

### Example

HPMBQ91 was first identified in a cDNA library from human placenta. The clone was identified as a novel human protein possessing homology to several previously described neurokinin precursor proteins. For example, HPMBQ91 contains an open reading frame (nucleotide number 1 to 360) encoding a polypeptide of 121 amino acids which has about 70% identity (using Smith-Waterman) in 121 amino acid residues with mouse neurokinin B precursor (K. Kako *et al.,* Biomed. Res. 14: 253-259, 1993). Alignment of the putative neuropeptide precursor suggested that the cDNA clone was full length. Following extensive sequencing of both the sense and the antisense strands of the clone, a full-length cDNA copy of HPMBQ91 was subsequently subcloned into bacterial and baculovirus expression vectors, pQE-9 (Qiagen, Inc., Chatsworth, CA) and pA2GP, respectively, for the production and purification of HPMBQ91 protein.

SEQ ID NO:1

-132 CT CCACT CGGTTT CT CT CTTT G CAGGAG CACCGG CAG CACCAGT GT GT GAGGGGAG CAGG
-72 CAG CGGT CCT AG CCAGTT CCTT GAT CCT G CCAGA CCACCCAG CCCCT GG CACAGAG CT G C
-12 T CCACAGG CACCAT GAGGAT CAT G CT G CT ATT CACAG CCAT CCT GG CCTT CAG CCT AG CT
49 CAGAG CTTT GGGG CT GT CT GT AAGGAG CCACAGGAGGAGGT GGTT CCT GG CGGGGG CCG C
109 AG CAAGAGGGAT CCAGAT CT CT ACCAG CT G CT CCAGAGA CT CTT CAAAAG CCACT CAT CT
169 CT GGAGGGATT G CT CAAAG CCCT GAG CCAGG CT AG CACAGAT CCT AAGGAAT CAACAT CT
229 CCCGAGAAA CGT GACAT G CAT GA CTT CTTT GT GGGA CTT AT GGG CAAGAGGAG CGT CCAG
289 CCAGA CT CT CCT ACGGAT GT GAAT CAAGAGAA CGT CCCCAG CTTT GG CAT CCT CAAGT AT
349 CCCCCGAGAG CAGAAT AGGT ACT CCA CTT CCGGA CT CCT GGA CT G CATT AGGAAGA CCT C
409 T TT CCCT GT CCCAAT CCCCAGGT G CG CACG CT CCT GTT ACCCTTT CT CTT CCCT GTT CTT
469 GT AACATT CT NGT G CTTT GA CT CCTT CT CCAT CTTTT CT ACCT GACCCT GGT GT GGAAA C
529 T G CAT AGT GAAT AT CCCCAACCCCAAT GGG CATT GA CT GT AGAAT ACCCT AGAGTT CCT G
589 T AGT GT CCT ACATT AAAAAT AT AAT GT CT CT CT CT ATT CCT CAACAAAT AAAGGATTT

SEQ ID NO:2

1 
61 
121 

SEQ ID NO:3

1 DPDLYQLLQ RLFK SH SSLEGLLKAL SQASTDPKE ST SPE 39

SEQ ID NO:4
1 DMHDFFVGLM 10

## Claims

1. An isolated polynucleotide comprising a nucleotide sequence that has at least 85% identity to a nucleotide sequence encoding the HPMBQ91 polypeptide of SEQ ID NO:2 over its entire length; or a nucleotide sequence complementary to said nucleotide sequence.

2. The polynucleotide of claim 1 wherein said nucleotide sequence comprises the HPMBQ91 polypeptide encoding sequence contained in SEQ ID NO:1.

3. An isolated polynucleotide comprising a nucleotide sequence wherein said nucleotide sequence is at least 85% identical to that contained in SEQ ID NO:1.

4. The polynucleotide of claim 3 which is the polynucleotide of SEQ ID NO: 1.

5. The polynucleotide of claim 1 which is DNA or RNA

6. A DNA or RNA molecule comprising an expression system, wherein said expression system is capable of producing a polypeptide comprising an amino acid sequence, which has at least 85% identity with the polypeptide of SEQ ID NO:2 when said expression system is present in a compatible host cell.

7. A host cell comprising the expression system of claim 6.

8. A process for producing a HPMBQ91 polypeptide comprising culturing a host of claim 7 under conditions sufficient for the production of said polypeptide and recovering the polypeptide from the culture.

9. A process for producing a cell which produces a HPMBQ91 polypeptide thereof comprising transform ing or transfecting a host cell with the expression system of claim 6 such that the host cell, under appropriate culture conditions, produces a HPMBQ91 polypeptide.

10. A HPMBQ91 polypeptide comprising an amino acid sequence which is at least 75% identical to the amino acid sequence of SEQ ID NO:2 over its entire length.

11. The polypeptide of claim 10 which comprises the amino acid sequence of SEQ ID NO:2.

12. The polypeptide of claim 10 which has the amino acid sequence of SEQ ID NO:2.

13. A HPMBQ91 peptide derived from the polypeptide of SEQ ID NO:2, excluding the peptide sequence of SEQ ID NO:4.

14. A HPMBQ91 peptide according to claim 13 comprising the sequence of SEQ ID NO:3.

15. A HPMBQ91 peptide according to claim 13 having the sequence of SEQ ID NO:3.

16. An antibody immunospecific for the HPMBQ91 polypeptide of claim 10.

17. A method for the treatment of a subject in need of enhanced activity or expression of HPMBQ91 polypeptide, or peptide derived therefrom, of any one of claims 10 to 15 comprising:
(a) administering to the subject a therapeutically effective amount of an agonist to said polypeptide; and/or
(b) providing to the subject an isolated polynucleotide comprising a nucleotide sequence that has at least 85% identity to a nucleotide sequence encoding the HPMBQ91 polypeptide of SEQ ID NO:2, or the peptide of SEQ ID NO:3, over its entire length; or a nucleotide sequence complementary to said nucleotide sequence in a form so as to effect production of said polypeptide or peptide activity *in vivo.*

18. A method for the treatment of a subject having need to inhibit activity or expression of HPMBQ91 polypeptide, or peptide derived therefrom, of any one of claims 10 to 15 comprising:
(a) administering to the subject a therapeutically effective amount of an antagonist to said polypeptide; and/or
(b) administering to the subject a nucleic acid molecule that inhibits the expression of the nucleotide sequence encoding said polypeptide; and/or
(c) administering to the subject a therapeutically effective amount of a polypeptide that competes with said polypeptide for its ligand, substrate , or receptor.

19. A process for diagnosing a disease or a susceptibility to a disease in a subject related to expression or activity of HPMBQ91 polypeptide of claim 10 in a subject comprising:
(a) determining the presence or absence of a mutation in the nucleotide sequence encoding said HPMBQ91 polypeptide in the genome of said subject; and/or
(b) analyzing for the presence or amount of the HPMBQ91 polypeptide expression in a sample derived from said subject.

20. A method for identifying compounds which inhibit (antagonize) or agonize the HPMBQ91 polypeptide of claims 10 to 12, or the peptide of any one of claims 13 to 15 which comprises:
(a) contacting a candidate compound with cells which express the HPMBQ91 polypeptide (or cell membrane expressing HPMBQ91 polypeptide) or respond to HPMBQ91 polypeptide; and
(b) observing the binding, or stimulation or inhibition of a functional response; or comparing the ability of the cells (or cell membrane) which were contacted with the candidate compounds with the same cells which were not contacted for HPMBQ91 polypeptide activity;
or
(c) contacting a candidate compound with cells which express the receptor for a peptide of claims 11 to 14 (or cell membrane expressing a receptor); and
(d) observing the stimulation or inhibition of binding of the peptide or the stimulation or inhibition of a functional response.

21. An agonist or antagonist identified by the method of claim 19.

22. An isolated HPMBQ91 polynucleotide comprising a nucleotide sequence selected from the group consisting of:
(a) a nucleotide sequence having at least 85% identity to a nucleotide sequence encoding the HPMBQ91 polypeptide expressed by the cDNA insert deposited at the ATCC with Deposit Number 97375; and
(b) a nucleotide sequence complementary to the nucleotide sequence of (a).
